# EUROPÄISCHE PATENTSCHRIFT

(11) **EP 0 630 954 B1**
(45) Veröffentlichungstag und Bekanntmachung des Hinweises auf die Patenterteilung: **08.01.1997**
(21) Anmeldenummer: 94107457.7
(22) Anmeldetag: 13.05.1994
(51) Int. Cl.: C09K 19/34, C09K 19/38, C07D 249/20, G03G 5/06

(54) **Niedermolekulare und polymere flüssigkristalline Benztriazole und deren Verwendung**
Low-molecular-weight and polymeric liquid cristalline benztriazoles and their use
Benztriazoles liquides cristallins, de faible poids moléculaire et polymères et leur utilsation

(30) Priorität: 21.05.1993 DE 4317093
(43) Veröffentlichungstag der Anmeldung: 28.12.1994
(73) Patentinhaber: BASF Aktiengesellschaft, 67063 Ludwigshafen (DE)
(72) Erfinder: Closs, Friedrich, Dr., D-68165 Mannheim (DE); Siemensmeyer, Karl, Dr., D-67227 Frankenthal (DE); Funhoff, Dirk, Dr,, D-69126 Heidelberg (DE)

(56) Entgegenhaltungen:
- EP-A- 0 093 330
- EP-A- 0 274 844
- EP-A- 0 418 386

## Beschreibung

Die Erfindung betrifft niedermolekulare und polymere flüssigkristalline 2-substituierte Benztriazole, ihre Verwendung als Photoleiter (Ladungsträger transportierende Verbindungen) in elektrophotographischen Aufzeichnungsmaterialien, sowie ihre Verwendung für Fluoreszenzdisplays aufgrund der starken Fluoreszenz dieser Flüssigkristalle.

Photoleiter sind eine interessante Materialklasse, die in Kopierern, Laserdruckern und Offset-Druckplatten in großem Umfang technisch eingesetzt werden.

Aus der EP-A 93 329 und EP-A 93 330 sind elektrophotographische Aufzeichnungsmaterialien bekannt, die als Photoleiter Triazole der Formel 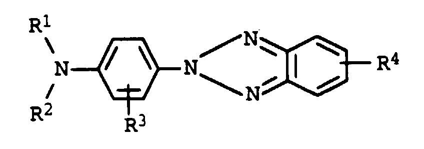 enthalten, worin R¹ und R² u.a. Wasserstoff, Alkyl, Allyl, Benzyl oder gegebenenfalls substituiertes Phenyl, R³ Wasserstoff, Alkyl, Alkoxy oder Halogen und R⁴ Wasserstoff, Alkyl, Alkoxy, Vinyl, Allyl, Dialkylamino, Nitro, Cyan oder Acryloyl bedeuten können.

Ein Nachteil der bisher verwandten Benztriazole ist ihre hohe Kristallisationsneigung in den meisten Bindemittelsystemen. Zur Herstellung von Photoleiterschichten werden daher häufig komplexe Mischungen von Photoleitern eingesetzt.

Ein genereller Nachteil organischer Photoleiter ist ihre geringe Ladungsträgerbeweglichkeit. Für eine Anwendung in schnellen Laserdruckern oder Kopierern muß daher bis dato auf anorganische Photoleiter, die toxisches Selen enthalten, zurückgegriffen werden.

Andererseits ist bekannt, daß hochgeordnete Systeme wie einkristallines Anthracen höhere Ladungsträgerbeweglichkeiten aufweisen. Es hat daher nicht an Versuchen gefehlt, die flüssigkristalline Ordnung in niedermolekularen oder polymeren Flüssigkristallen auszunutzen um zu schnelleren Photoleitern zu gelangen. Kürzlich gelang der Nachweis einer erhöhten Photoleitung in diskotischer (vgl. DE-A 41 26 496) und nematischer Phase (vgl. DE-A-42 11 087).

Aufgabe der vorliegenden Erfindung ist es, Benztriazole mit flüssigkristallinen Eigenschaften bereitzustellen, insbesondere solche, die photoleitend sind und in flüssigkristalliner Phase eine höhere Photoleitfähigkeit aufweisen als in nichtflüssigkristalliner Phase. Außerdem ist von Interesse, ob die starke Fluoreszenz der 2-Aminophenylbenztriazole auch bei den flüssigkristallinen Vertretern gewahrt bleibt.

Überraschenderweise zeigen bestimmte 2-substituierte Benztriazole flüssigkristallines Verhalten. Trotz Abweichung von der ideal linearen Stäbchenform weisen die Benztriazole nematische und smektische Phasen mit z.T. großer Phasenbreite auf. Einige Vertreter sind zudem gute Photoleiter und zeigen in flüssigkristalliner Phase eine höhere Photoleitfähigkeit als in isotroper oder polykristalliner Phase. Ferner zeichnen sich die erfindungsgemäßen Benztriazole durch eine hohe Fluoreszenz aus.

Gegenstand der vorliegenden Erfindung sind flüssigkristalline 2-substituierte Benztriazole der allgemeinen Formel (I) 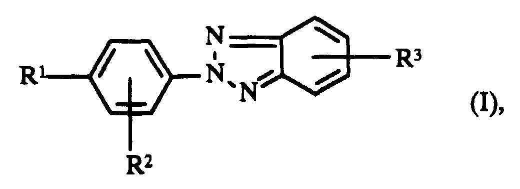 worin
- R¹: für NR⁴R⁵ oder 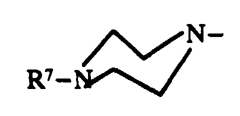 steht, wobei
- R⁴: H, C₁-C₁₂-Alkyl,
- R⁵: C₅-C₁₂-Alkyl und
- R⁷: C₅-C₁₂-Alkyl, C₅-C₁₂-Acyl, Vinyl, C₃-C₆-Alkenyl, Acryl oder Methacryl sein können,
- R²: für H, C₁-C₁₂-Alkyl, C₁-C₁₂-Alkoxy, Hydroxy oder Halogen steht und
- R³: für 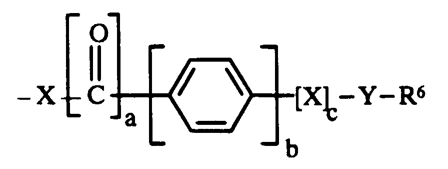 steht, wobei X = 0 oder S; a = 0 oder 1; b = 0, 1 oder 2; c = 0 oder 1 sein können, jedoch a, b und c nicht gleichzeitig 0 sein dürfen und wobei Y = C₂-C₁₁-Alkylen oder Oxaalkylen mit 1 bis 5 Sauerstoffatomen und 3 bis 12 Kohlenstoffatomen und R⁶ = -CH₃, -OCH₃, -CH=CH₂, -O-CH=CH₂, 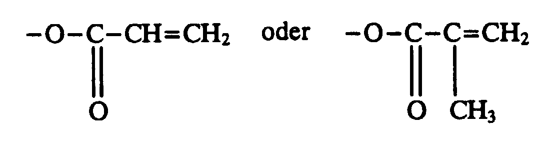 sein können mit der Maßgabe, daß für den Fall, daß
- R¹ =: 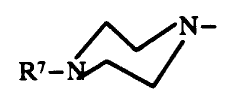 ist,
- R³: die Bedeutung H, C₁-C₁₂-Alkoxy, C₁-C₁₂-Acyloxy, Nitro, Cyan, Dialkylamino und Acryloyl oder Methacryloyl haben kann.

Gegenstand der vorliegenden Erfindung sind auch polymere, einen Polymerisationsgrad von 2 bis 100 aufweisende Benztriazole, hergestellt aus monomeren Einheiten der allgemeinen Formel (II) 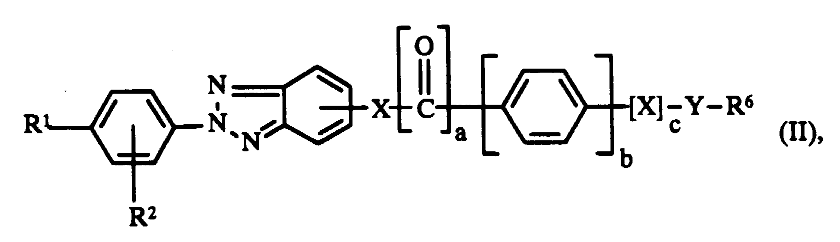 worin R¹, R², X, Y, a, b und c die in Anspruch 1 angegebene Bedeutung haben und R⁶ für einen polymerisierbaren Rest aus der Gruppe -CH=CH₂, -O-CH=CH₂, 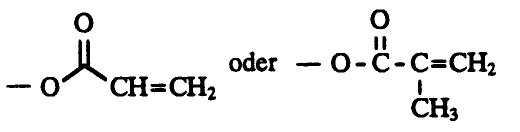 steht.

Gegenstand der vorliegenden Erfindung sind außerdem elektrophotographische Aufzeichnungsmaterialien, bestehend aus einem elektrisch leitenden Schichtträger, einer ladungsträgererzeugenden Sensibilisatorschicht und einer photoleitfähigen Schicht, wobei in der photoleitfähigen Schicht ein oder mehrere niedermolekulare oder polymere 2-substituierte Benztriazole gemäß der Formel (I) bzw. (II) enthalten sind.

Gegenstand der vorliegenden Erfindung ist auch die Verwendung der flüssigkristallinen 2-substituierten Benztriazole für Fluoreszenzdisplays.

Die Erhöhung der Photoleitfähigkeit in erfindungsgemäßen Benztriazolen mit kalamitischen Eigenschaften kann auch durch eine homogen planare Orientierung der Ladungsträgermoleküle erreicht werden, wobei die Ladungsträgermoleküle durch mechanisches Scheren oder Strecken, durch Anlegen eines elektrischen oder magnetischen Feldes oder durch thermische Behandlung orientiert werden können.

Erfindungsgemäße niedermolekulare flüssigkristalline 2-substituierte Benztriazole sind solche der allgemeinen Formel (I) 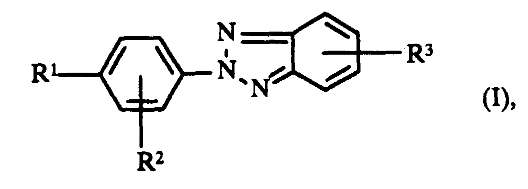 worin
- R¹: für NR⁴R⁵ oder 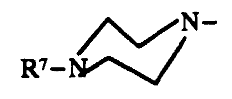 steht, wobei
- R⁴: H, C₁-C₁₂-Alkyl, vorzugsweise C₁-C₅-Alkyl, beispielsweise Methyl, Ethyl, Propyl, Butyl, Pentyl und
- R⁵: C₅-C₁₂-Alkyl, vorzugsweise C₆-C₁₂-Alkyl, beispielsweise Hexyl und Octyl,
- R⁷: C₅-C₁₂-Alkyl, vorzugsweise C₅-C₈-Alkyl, z.B. Pentyl, C₅-C₁₂-Acyl, wie z.B. Valeryl, Capryl, Vinyl, C₃-C₆-Alkenyl, Acryl oder Methacryl sein können,
- R²: für H, C₁-C₁₂-Alkyl, C₁-C₁₂-Alkoxy, Hydroxy oder Halogen wie z.B. F, Cl oder Br steht und
- R³: für 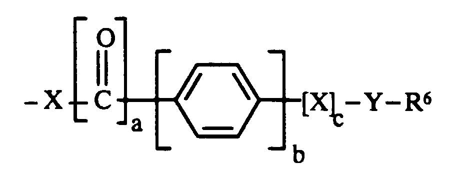 steht, wobei X = 0 oder S; a = 0 oder 1; b = 0, 1 oder 2; c = 0 oder 1 sein können, jedoch a, b und c nicht gleichzeitig 0 sein dürfen und wobei Y = C₂-C₁₁-Alkylen oder Oxaalkylen mit 1 bis 5 Sauerstoffatomen und 3 bis 12 Kohlenstoffatomen und R⁶ = -CH₃, -OCH₃, -CH=CH₂, -O-CH=CH₂, 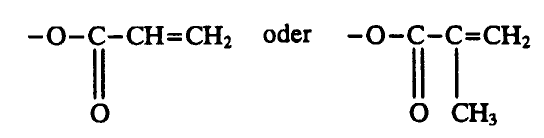 sein können mit der Maßgabe, daß für den Fall, daß
- R¹ =: 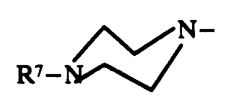 ist,
- R³: die Bedeutung H, C₁-C₁₂-Alkoxy, wie z.B. Methoxy, Ethoxy, Propoxy, Butoxy, C₁-C₁₂-Acyloxy, wie z.B. Acetoxy, Valeryl, Capryl, Nitro, Cyan, Dialkylamino, wie z.B. Dimethylamino, Diethylamino, Dibutylamino und Acryloyl oder Methacryloyl haben kann.

Die erfindungsgemäßen 2-substituierten Benztriazole sind nach bekannten Methoden der organischen Chemie darstellbar. Sie können beispielsweise erhalten werden aus ortho-Nitroazoverbindungen der allgemeinen Formel (III) 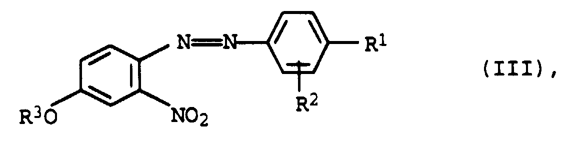 worin R¹, R² und R³ die obengenannte Bedeutung haben, nach den in Houben-Weyl, Methoden der organischen Chemie, 4. Auflage, Band X/3, S. 425 ff., beschriebenen Methoden. Verbindungen der allgemeinen Formel (III) können nach Standardverfahren hergestellt werden. Ausgehend von 5-Methoxybenztriazolen sind durch Etherspaltung in mit Bromwasserstoff gesättigtem Wasser - wie in den Beispielen beschrieben - 5-Hydroxybenztriazole zugänglich.

Durch Veretherung mit geeigneten Alkoholen oder Veresterung mit geeigneten Säuren oder Säurechloriden sind nach Standardverfahren viele derartiger flüssigkristalliner Benztriazole einfach zugänglich.

Erfindungsgemäße polymere flüssigkristalline Benztriazole sind solche, die über einen flexiblen Spacer an eine Polymerkette gebunden ein Benztriazol der allgemeinen Formel (I) enthalten.

Die polymere Anbindung kann sowohl an der Hydroxygruppe in 5-Stellung des Benztriazols als auch am terminalen Stickstoff des 2-Aminophenyl- oder 2-Pyrazinylphenyl-Restes erfolgen. Aus den entsprechenden Monomer-Vorstufen lassen sich nach Standardverfahren Polyacrylate, Polymethacrylate, Polysiloxane und Polyvinylether herstellen. Ein Beispiel für ein polymeres, flüssigkristallines Benztriazol ist z.B.

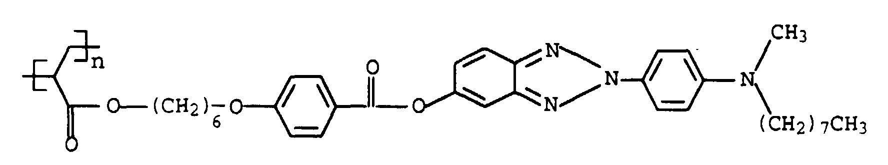

Für die Verwendung der erfindungsgemäßen flüssigkristallinen 2-substituierten Benztriazole für elektrophotographische Aufzeichnungsmaterialien können zur Erhöhung der Lichtempfindlichkeit der Schichten Sensibilisatoren, d.h. Ladungsträger erzeugende Verbindungen zugesetzt werden. Derartige Verbindungen sind z.B. die aus DE-A 22 37 399 und DE-A 31 10 955 bekannten Perylentetracarbonsaurederivate. Besonders bevorzugt sind Sensibilisatoren wie Rhodamin B (C.I. 45170) und Astrazonorange (C.I. 48035).

Die erfindungsgemäßen Photoleiter werden in der Regel in Form von dünnen photoleitenden Schichten verwendet, wobei auch eine Trennung des Ladungstransports von der Ladungserzeugung im Sinne einer Zweischichten-Anordnung möglich sein kann, wie sie in der Elektrophotographie angewandt wird. Hierbei befindet sich der erfindungsgemäße Photoleiter in der photoleitfähigen Ladungstransportschicht, an die eine übliche und bekannte Ladungsträger erzeugende Sensibilisatorschicht angrenzt. Die Aufladung erfolgt hierbei in der Regel durch eine Hochspannungscorona.

Die Erzeugung der erfindungsgemäßen Schichten auf einer Trageroberfläche kann durch Auftragen einer Schmelze oder in üblicher und bekannter Weise z.B. durch Aufrakeln einer Lösung der Verbindungen auf eine Trägeroberfläche erfolgen. Dabei kannen der Lösung verschiedene Hilfsstoffe, z.B. zur Verbesserung der Verlaufeigenschaften, zugesetzt werden.

Als Lösungsmittel können beispielsweise Tetrahydrofuran oder Dichlormethan verwendet werden.

Diese photoleitenden Schichten haben im allgemeinen eine Schichtdicke zwischen 1 und 100, vorzugsweise zwischen 1 und 50 und besonders bevorzugt zwischen 1 und 30 µm.

Die Photoleiter bzw. die photoleitenden Schichten können zwischen leitfähig beschichteten, transparenten Substraten angeordnet werden, für die Glasplatten oder Platten aus optisch transparenten Kunststoffen (beispielsweise Polymethylmethacrylat, Polycarbonat etc.) verwendet werden. Die leitfähige Beschichtung des Substrats kann aus elektrisch leitfähigen Polymeren, Halbleitern oder Metallen wie z.B. Aluminium, Silber oder Gold bestehen. Die Dicke der Beschichtung ist hierbei allerdings so zu wählen, daß die optische Durchlässigkeit nicht zu sehr beeinträchtigt wird. Besonders vorteilhafte Beschichtungen bestehen aus ITO ("indium tin oxide").

Zur Erzeugung eines Photostromes wird hierbei an die elektrisch leitfähig beschichteten Platten im allgemeinen eine Gleichspannung zwischen 5 und 50 V angelegt.

Der flüssigkristallin kalamitische Zustand, in dem die Photoleitfähigkeit höher ist als im ungeordneten, isotropen Zustand kann vorteilhafterweise durch eine homogen planare Orientierung eingestellt werden. Die Orientierung der Ladungsträger kann z.B. mechanisch (durch Strecken oder Scheren) oder durch elektrische oder magnetische Felder erreicht werden. Die Einstellung einer Orientierung ist auch mittels orientierender Unterschichten, die z.B. Polyimide enthalten oder aus diesen bestehen, möglich. Die einfachste Möglichkeit ist eine thermische Behandlung (Tempern).

Die erfindungsgemäßen Photoleiter und photoleitfähigen Filme können in der Elektrophotographie, in Laserdruckern, im Offsetdruck, oder aber in der Mikroelektronik für lichtempfindliche Schalter verwandt werden. Darüberhinaus kann die Erfindung in all den Bereichen eingesetzt werden, in denen die Erhöhung der Photoleitfähigkeit durch molekulare Ordnung ausgenutzt wird.

Die Erfindung wird durch die nachfolgenden Beispiele erläutert.

Die in den Beispielen genannten Teile und Prozente sind, soweit nicht anders angegeben, Gewichtsteile bzw. Gewichtsprozente.

Bei den Angaben über das Phasenverhalten bedeutet
- k: kristalline Phase,
- S_{A}: smektisch A-Phase,
- n: nematische Phase,
- LC: nicht identifizierte flüssigkristalline Phase,
- i: isotrope Schmelze.

### Beispiel 1

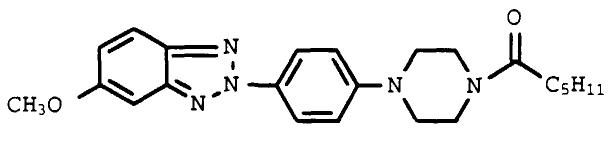

36 g 4-Methoxy-2-Nitro-4'N-Caproylpyrazinylazobenzol und 97 g Triethylphosphit wurden 4 h unter Rückfluß zum Sieden erhitzt. Nach dem Abkühlen wurde abgesaugt und aus Ethanol umkristallisiert.

Es wurden so 23 g gelbe Kristalle erhalten.

Phasenverhalten: k 197,9°C S_{A} 201,7°C i
C₂₃H₂₉N₅O₂

| Analyse: | | | | |
|---|---|---|---|---|
| | C | H | N | O |
| ber. | 67,8 | 7,2 | 7,9 | 17,2 |
| gef. | 67,8 | 7,3 | 8,1 | 17,4 |

### Beispiel 2

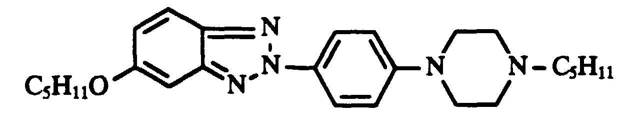

a) 20 g 5-Methoxy-2(4-Caproylpyrazinylphenyl)benztriazol (Bsp. 1) wurden mit 112 g 47 %iger HBr in Wasser 6 h auf 120 °C erhitzt. Der farblose Festkörper wurde abgesaugt, mit Wasser neutralgewaschen und im Vakuum bei 40 °C getrocknet.
   Rohausbeute: 19 g 5-Hydroxy-2-(4-Pyrazinylphenyl)benztriazol-Hydrobromid.
b) 17 g des Hydrobromids wurden mit 24,4 g Pentylbromid, 6 g KOH und 118 g EtOH/5 g H₂O 6 h unter Rückfluß zum Sieden erhitzt. Der Ansatz wurde auf 100 g H₂O gegossen, abgesaugt und im Vak. bei 50 °C getrocknet. Es wurden 19,8 g gelbe Kristalle erhalten (umkristallisiert aus Essigester)

Phasenverhalten: k 102°C S_{A} 151°C i
C₂₆H₃₇N₅O

| Analyse: | | | | |
|---|---|---|---|---|
| | C | H | N | O |
| ber. | 71,7 | 8,6 | 16,1 | 3,7 |
| gef. | 71,5 | 8,7 | 15,7 | 3,9 |

### Beispiel 3

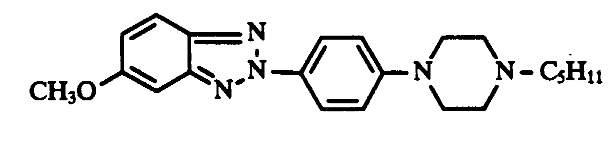

1,3 g 4-Methoxy-2-Nitro-4'N-Pentylpyrazinylazobenzol und 19 g Triethylphosphit wurden 3 h unter Sieden zum Rückfluß erhitzt. Es wurde abgekühlt. Dabei wurden 0,5 g gelbe Kristalle gewonnen.

Phasenverhalten: k 113°C S_{A} 190,8°C n 191,7°C i
C₂₂H₂₉N₅O

| Analyse: | | | | |
|---|---|---|---|---|
| | C | H | N | O |
| ber. | 69,6 | 7,7 | 18,5 | 4,2 |
| gef. | 69,4 | 7,8 | 18,3 | 4,5 |

### Beispiel 4

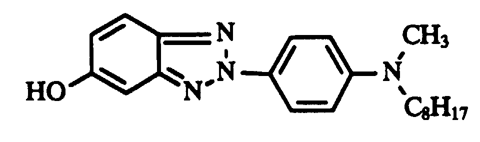

40 g 5-Methoxy-2-[4-(Methyl-n-Octylamino)phenyl]benztriazol wurden mit 440 g 47 %igem HBr in H₂O 6 h unter Rückfluß zum Sieden erhitzt. Es wurde abgekühlt, mit Wasser neutralgewaschen und im Vakuum bei 50°C getrocknet.

Ausbeute: 405, g 5-Hydroxy-2-[4-(Methyl-n-Octylamino)phenyl]-benztriazol-Hydrobromid.

### Beispiel 5

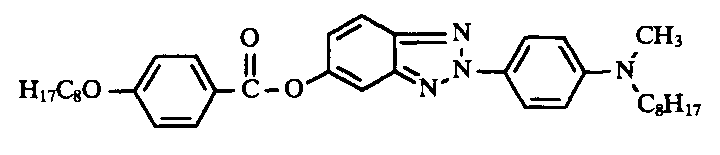

8,7 g 5-Hydroxy-2-[4-(Methyl-n-Octylamino)phenyl]benztriazol-Hydrobromid und 8,6 g 4-Octyloxybenzoylchlorid wurden in 29 g Pyridin 5 h unter Rückfluß zum Sieden erhitzt. Der Ansatz wurde auf Raumtemperatur abgekühlt, filtriert und auf 100 g verdünnte HCl gegossen. Die gelben Kristalle wurden abgesaugt.

Ausbeute: 7 g C₃₆H₄₈N₄O₃

Phasenverhalten: Monotroper Flüssigkristall

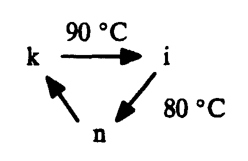

### Beispiel 6

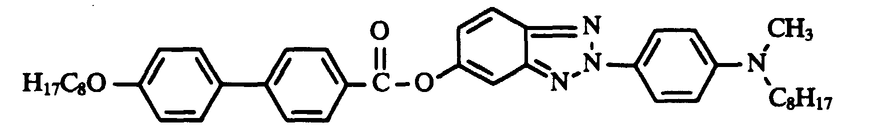

2,4 g 4-Octyloxy-4'-Chlorcarbonylbiphenyl und 4,3 g 5-Hydroxy-2-[4-(Methyl-n-Octylamino)phenyl]benztriazol-Hydrobromid wurden in 40 g Pyridin 6 h unter Sieden zum Rückfluß erhitzt. Der Ansatz wurde abgekühlt und auf 200 g verdünnte HCl gegeben. Die hellgelben Kristalle wurden abgesaugt und mit Wasser gewaschen.

Ausbeute: 3 g (umkristallisiert aus Essigester) C₃₆H₄₈N₄O₃
Phasenverhalten k 181°C n 228,4°C i

### Beispiel 7

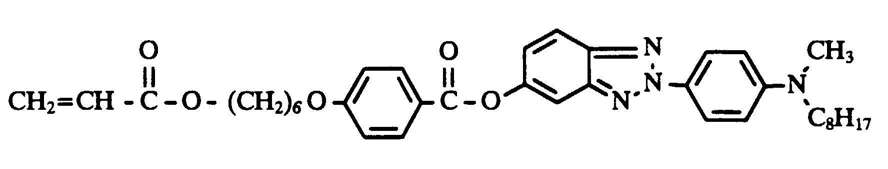

5,6 g 5-Hydroxy-2-[4-(Methyl-n-octylamino)phenyl]benztriazol-Hydrobromid, 5,0 g 4-Acryloyloxyhexoxybenzoesäure und 0,3 g 4-Pyrrolidinopyridin wurden in 250 g trockenem Methylenchlorid gelöst. Bei 0 bis + 5 °C wurden 6 g Dicyclohexylcarbodiimid in 125 g trockenem Methylenchlorid zugetropft. Dann wurde 18 h bei Raumtemperatur gerührt und von unlöslichem Rückstand abgetrennt. Das Lösungsmittel wurde im Vakuum eingeengt und der Rückstand aus Ethanol umkristallisiert.

Ausbeute: 7 g C₃₇H₄₆N₄O₅

Im Bereich des Schmelzpunktes (80°C) tritt Polymerisation auf.

### Beispiel 8

3,8 g Monomer von Beispiel 7 wurden in 88 g trockenem THF gelöst. Es wurden 0,4 g Porofor N zugegeben, mit Argon gespült und 100 h bei 50°C gerührt. Nach dem Abfiltrieren wurde das Filtrat mit Methanol versetzt. Das Polymer ließ sich so als Öl abscheiden und wurde mehrfach aus THF/ Methanol umgefällt.

Ausbeute: 3,3 g
DSC g 48°C n 65°C i (DSC = Differentialthermoanalyse; g = Glasphase)
Polymerisationsgrad: 5-6
(nach GPC = Gelpermeationschromatographie)

### Beispiel 9

### Beispiel zur Photoleitung in flüssigkristalliner Phase von flüssigkristallinen Benztriazolderivaten

Zur Bestimmung des Photostromes in flüssigkristalliner Phase wurde eine 1:1 Mischung der Substanzen von Beispiel 5 und 6 durch Ineinanderschmelzen hergestellt. Die Phasenumwandlungstemperaturen dieser Mischung betrugen:
k 60°C LC 145°C i.

Die zu untersuchende Mischung wurde in einer Meßzelle bestehend aus zwei Glasplatten, die eine transparente, leitende Schicht aus Indium-Zinn-Oxid besaßen, präpariert. Die Schichtdicke der Probe betrug ca. 10 µm, welche durch eine Polyester-Folie von 8 µm eingestellt war. Die Elektrodenfläche betrug 4 mm². An die Probe wurde ein Gleichspannungssignal von 9 V angelegt, was einer angelegten Feldstärke von ca. 900 kV m⁻¹ entspricht. Die Beleuchtung der Probe erfolgte mit einer Kaltlichtquelle. Der anfallende Lichtstrahl wurde durch einen Chopper ("Lichtzerhacker") mit einer Frequenz von 10 Hz moduliert, d.h. in Lichtpulse von 50 msec Länge bei ebensolanger Dunkelphase zerlegt. Die Detektion des Photostromes erfolgte über den Spannungsabfall an einem Widerstand von 182.000 Ω mittels eines Lock-In-Verstärkers (EG&G 5210). Um die Probe temperaturabhängig untersuchen zu können, wurde sie in einem Heiztisch (Mettler FP 80/82) temperiert.

Für obige Mischung wurden folgende Werte erhalten:

| Temperatur [°C] | Photostrom [pA] | Phase |
|---|---|---|
| 40 | 5 | kristallin |
| 70 | 15 | flüssigkristallin |
| 100 | 50 | flüssigkristallin |
| 110 | 70 | flüssigkristallin |
| 130 | 130 | flüssigkristallin |
| 150 | 65 | isotrop |

Wie sich aus der Tabelle ergibt, zeigte die Probe in der kristallinen Phase nahezu keinerlei Photostrom. In der flüssigkristallinen Phase steigt dieser stark an, um dann zur isotropen Phase hin wieder abzufallen.

## Patentansprüche

1. 2-substituierte Benztriazole der allgemeinen Formel (I) 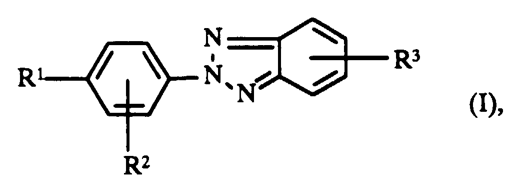 in der
R¹ für NR⁴R⁵ oder 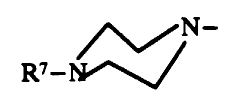 steht, wobei
R⁴ H, C₁-C₁₂-Alkyl,
R⁵ C₅-C₁₂-Alkyl und
R⁷ C₅-C₁₂-Alkyl, C₅-C₁₂-Acyl, Vinyl, C₃-C₆-Alkenyl, Acryl oder Methacryl sein können,
R² für H, C₁-C₁₂-Alkyl, C₁-C₁₂-Alkoxy, Hydroxy oder Halogen steht und
R³ für 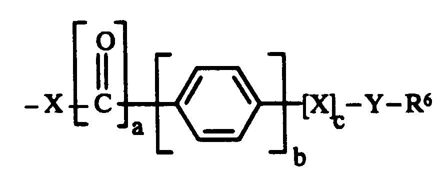 steht, wobei X = 0 oder S; a = 0 oder 1; b = 0, 1 oder 2; c = 0 oder 1 sein können, jedoch a, b und c nicht gleichzeitig 0 sein dürfen und wobei Y = C₂-C₁₁-Alkylen oder Oxaalkylen mit 1 bis 5 Sauerstoffatomen und 3 bis 12 Kohlenstoffatomen und R⁶ = -CH₃, -OCH₃, -CH=CH₂, -O-CH=CH₂, 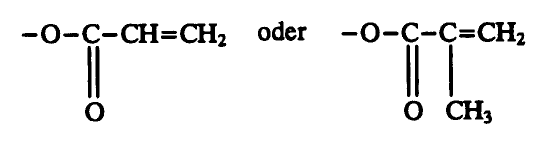 sein können mit der Maßgabe, daß für den Fall, daß
R¹ = 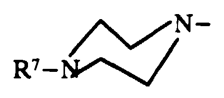 ist,
R³ die Bedeutung H, C₁-C₁₂-Alkoxy, C₁-C₁₂-Acyloxy, Nitro, Cyan, Dialkylamino und Acryloyl oder Methacryloyl haben kann.

2. Flüssigkristalline polymere, einen Polymerisationsgrad von 2 bis 100 aufweisende Benztriazole, hergestellt aus monomeren Einheiten der allgemeinen Formel (II) 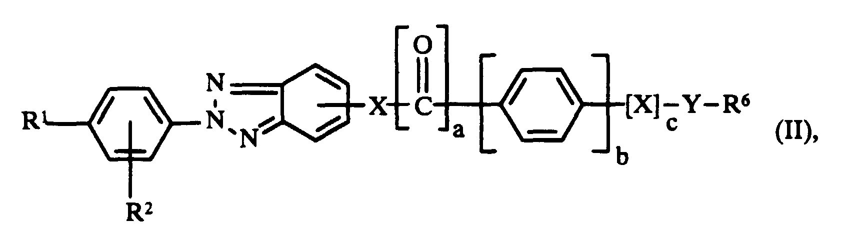 worin R¹, R², X, Y, a, b und c die in Anspruch 1 angegebene Bedeutung haben und R⁶ für einen polymerisierbaren Rest aus der Gruppe -CH=CH₂, -O-CH=CH₂, 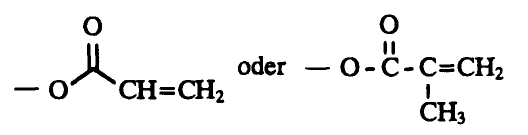 steht.

3. Elektrophotographisches Aufzeichnungsmaterial, bestehend aus einem elektrisch leitenden Schichtträger, einer ladungsträgererzeugenden Sensibilisatorschicht und einer photoleitfähigen Schicht, dadurch gekennzeichnet, daß es in der photoleitfähigen Schicht einen oder mehrere 2-substituierte Benztriazole nach einem der Ansprüche 1 oder 2 enthält.

4. Verwendung der flüssigkristallinen 2-substituierten Benztriazole nach einem der Ansprüche 1 oder 2 für Fluoreszenzdisplays.

## Claims

1. A 2-substituted benzotriazole of the formula (I) 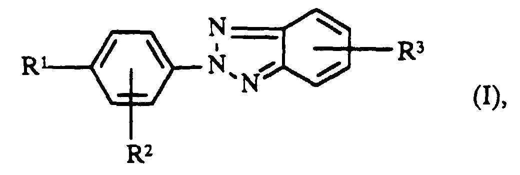 where
R¹ is NR⁴R⁵ or 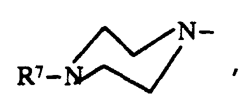 where
R⁴ is H or C₁-C₁₂-alkyl,
R⁵ is C₅-C₁₂-alkyl and
R⁷ is C₅-C₁₂-alkyl, C₅-C₁₂-acyl, vinyl, C₃-C₆-alkenyl, acryloyl or methacryloyl,
R² is H, C₁-C₁₂-alkyl, C₁-C₁₂-alkoxy, hydroxyl or halogen, and
R³ is 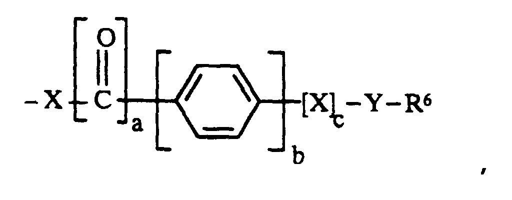 where X is O or S, a is 0 or 1, b is 0, 1 or 2, and c is 0 or 1, but where a, b and c must not simultaneously be 0 and where Y is C₂-C₁₁-alkylene or oxaalkylene having 1 to 5 oxygen atoms and 3 to 12 carbon atoms, and R⁶ is -CH₃, -OCH₃, -CH=CH₂, -O-CH=CH₂, 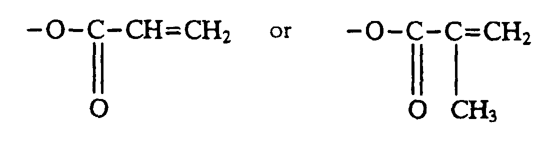
with the proviso that, in the case where
R¹ is 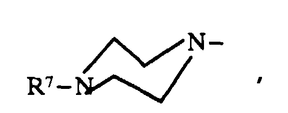
R³ is H, C₁-C₁₂-alkoxy, C₁-C₁₂-acyloxy, nitro, cyano, dialkylamino, acryloyl or methacryloyl.

2. A liquid-crystalline, polymeric benzotriazole having a degree of polymerization of from 2 to 100, prepared from monomeric units of the formula (II) 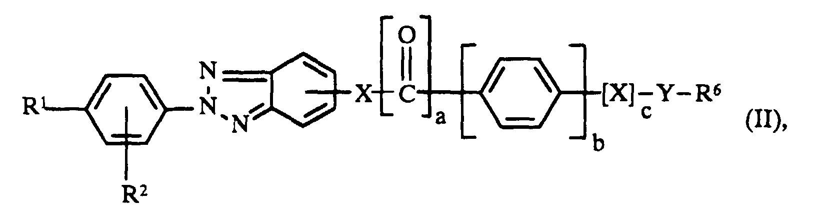 where R¹, R², X, Y, a, b and c are as defined in claim 1, and R⁶ is a polymerizable radical from the group consisting of -CH=CH₂, -O-CH=CH₂,

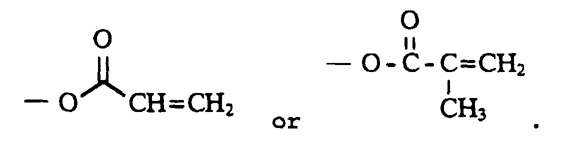

3. An electrophotographic recording material comprising an electroconductive layer support, a charge carrier-producing sensitizer layer and a photoconductive layer, wherein the photoconductive layer contains one or more 2-substituted benzotriazoles as claimed in claim 1 or 2.

4. The use of a liquid-crystalline, 2-substituted benzotriazole as claimed in claim 1 or 2 for fluorescent displays.

## Revendications

1. Benzotriazoles 2-substitués de formule générale (I) 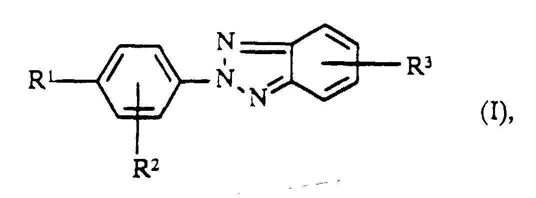 dans laquelle
R¹ est mis pour NR⁴R⁵ ou 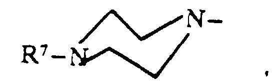 où
R⁴ peut être un atome d'hydrogène ou un reste alkyle en C₁-C₁₂,
R⁵ peut être un reste alkyle en C₅-C₁₂ et
R⁷ peut être un reste alkyle en C₅-C₁₂, acyle en C₅-C₁₂, vinyle, alcényle en C₃-C₆, acryle ou méthacryle,
R² est mis pour un atome d'hydrogène, un reste alkyle en C₁-C₁₂, alcoxy en C₁-C₁₂, hydroxy ou un atome d'halogène et
R³ est mis pour 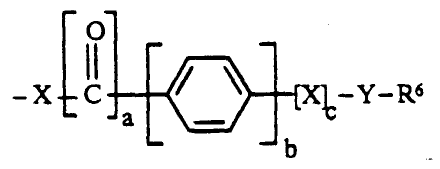 où X = O ou S; a = 0 ou 1; b = 0, 1 ou 2; c = 0 ou 1, mais a, b et c ne peuvent pas être mis pour 0 en même temps, et où Y = reste alkylène en C₂-C₁₁ ou oxaalkylène à 1-5 atomes d'oxygène et 3-12 atomes de carbone et R⁶ = -CH₃, -OCH₃, -CH=CH₂, -O-CH=CH₂, 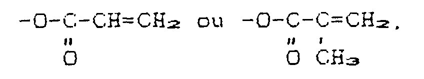
étant spécifié que dans le cas où
R¹ = 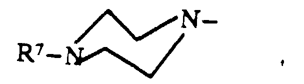
R³ peut representer un atome d'hydrogène, un reste alcoxy en C₁-C₁₂, acyloxy en C₁-C₁₂, nitro, cyano, dialkylamino, acryloyle ou méthacryloyle.

2. Benzotriazoles polymères cristallins liquides, ayant un degré de polymérisation de 2 à 100, préparés à partir de motifs monomères de formule générale (II) 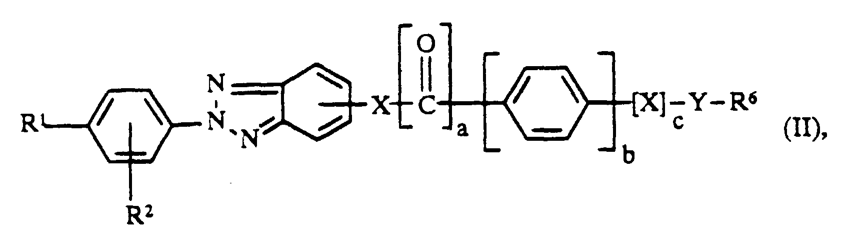 dans laquelle R¹, R², X, Y, a, b et c ont les significatiions données dans la revendication 1, et R⁶ est mis pour un reste polymérisable choisi dans le groupe constitué par -CH=CH₂, -O-CH=CH₂,

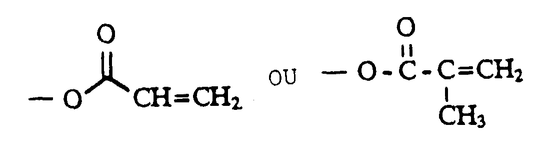

3. Matériel d'enregistrement électrophotographique, se composant d'un support de couches électroconducteur, d'une couche de sensibilisateur produisant des porteurs de charge et d'une couche photoconductrice, caractérisé en ce qu'il contient, dans la couche photoconductrice, un ou plusieurs benzotriazoles 2-substitué selon la revendication 1 ou 2.

4. Utilisation des benzotriazoles 2-substitués cristallins liquides selon la revendication 1 ou 2 pour des panneaux d'affichage fluorescents.
